# EUROPEAN PATENT APPLICATION

(11) **EP 1 698 696 A1**
(43) Date of publication of application: **06.09.2006**
(21) Application number: 04819752.9
(22) Date of filing: 16.11.2004
(51) Int. Cl.: C12N 15/12, C07K 14/47, A61K 31/426, A61K 31/4439, A61K 45/00, A61P 3/10, A61P 43/00, C07D 277/34, C07D 417/12, G01N 33/15, G01N 33/50

(54) **TARGET PROTEIN OF ANTIDIABETIC AGENT AND NOVEL ANTIDIABETIC AGENT INSUFUL CORRESPONDING THERETO**

(30) Priority: 01.12.2003 JP 2003402164
(71) Applicant: Reverse Proteomics Research Institute Co., Ltd, Kisarazu-shi, Chiba 292-0818 (JP)
(72) Inventor: SUWA, Yorimasa c/o Reverse Proteomics, Kisarazu-shi, Chiba 2920818 (JP); YAMADA, Tsuyoshi, c/o Sumitomo Pharma Co.,Ltd.., Osaka-shi, Osaka 554-0022 (JP); OSAKI, Hironori c/o Tsukuba R.L., Nippon Shinyaku, Tsukuba-shi, Ibaraki 305-0003 (JP); FURUYA, Minoru c/o Pharm.Disc.Res.Lab., Teijin Ph., Hino-shi, Tokyo 191-8512 (JP)
(74) Representative: Denison, Christopher Marcus
(86) International application number: PCT/JP2004/016996
(87) International publication number: WO 2005/054468

(57) **Abstract**

The present invention is intended to elucidate a molecular target of an antidiabetic such as a thiazolidine derivative. The present invention provides a screening method for an antidiabetic, comprising the steps of: bringing a candidate substance to be screened into contact with a protein represented by the following (a) or (b): (a) a protein comprising the amino acid sequence represented by SEQ ID NO: 2; or (b) a protein comprising an amino acid sequence derived from the amino acid sequence represented by SEQ ID NO: 2 with the deletion, substitution, addition, or insertion of one or plural amino acids and interacting with the antidiabetic; and detecting the interaction between the candidate substance and the protein.

## Description

### Technical Field

The present invention relates to a target protein of an antidiabetic known in the art and a screening method for a novel antidiabetic using the protein.

### Background Art

According to the WHO estimations, patients with diabetes are now (the year 2003) 150 •million people worldwide and are said to reach 300 million people in 2025. In the current United States, 6% of its population suffers from diabetes, and the related medical expenses including the cost of fighting complications caused by diabetes reached 98 billion dollars in 1997. The global market for oral hypoglycemic agents is estimated to be 800 billion to 900 billion yen and is also estimated to reach 2 trillion to 3 trillion yen in 2010.

In Japan, patients with diabetes were 6.9 million people in the 1998 survey and reached 13.7 million people in combined total with persons who exhibit reduced insulin efficacy and impaired glucose tolerance, alleged pre-diabetes. Drug therapy for diabetes utilizes injection preparations such as insulin as well as oral drugs, which are estimated to be a total of 180 billion to 210 billion yen.

There are 2 forms of diabetes: insulin-dependent diabetes (type 1), which is developed by significantly reduced insulin secretion; and insulin-independent diabetes (type 2), in which insulin secretion is maintained at some level but sill insufficient. Particularly, the number of patients with type 2 diabetes has significantly increased, and type 2 diabetes is said to affect 10% of adults aged 40 years and older. In our country, patients with this type 2 diabetes account for 90% or more of patients with diabetes. Type 2 diabetes is characterized by clinical conditions attributed to deficient insulin secretion and insulin resistance. Deficiencies in insulin action in type 2 diabetes are caused by the following pathogenesis:
· insufficient insulin secretion from pancreatic β-cells;
· excessive glucose release from the liver; and
· insulin resistance in peripheral tissues such as muscular and adipose tissues.

Insulin resistance is highly involved in the development of type 2 diabetes. Besides, its relationship with hypertension, obesity, hyperlipemia, and so on, is also pointed out. Recently, the prevention and treatment of diabetes place special emphasis on insulin resistance. Insulin resistance means a state of an inability of insulin, if present in the blood, to exert sufficient action in its target tissues such as hepatic, muscular, and adipose tissues. Persons having insulin resistance require more than normal amounts of insulin, because they possess reduced insulin sensitivity and inhibited normal insulin action. Insulin sensitivity is reported to decrease by 30 to 40% in nonobese patients with essential hypertension and decreases further in obese patients with essential hypertension. Thus, insulin resistance is found in allegedly 50 to 60% of the total cases of essential hypertension, 70 to 80% of cases having obesity, and 80% of cases having high neutral fat levels at fasting.

Insulin resistance is associated with environmental factors such as obesity, diabetes (particularly, insulin-independent diabetes with obesity), overeating, physical inactivity, stress, pregnancy, infectious diseases, aging, and the long-term use of steroid, in addition to genetic factors. Insulin resistance is attributed to the greatly decreased number of cell-surface insulin receptors, although they have normal insulin-binding ability, and reduced tyrosine kinase activity, due to insulin receptor gene abnormality and so on. In some cases, autoantibodies against insulin receptors are developed and, consequently, insulin resistance may be caused.

The manifestation of insulin resistance is seen in the form of insulin hypersecretion from pancreatic β-cells. Namely, organisms secrete insulin in large amounts for overcoming insulin resistance and therefore lead to elevated insulin levels in the blood. As a result, hyperinsulinemia is universally observed in most of them.

If insulin resistance is added, compensatory increased insulin secretion masks deficiencies in insulin action as long as pancreatic β-cells have sufficient reserve to secrete insulin. However, insufficient insulin secretion resulting from a disorder, if any, in pancreatic β-cells makes this compensation difficult and insulin action deficient, leading to hyperglycemia. This hyperglycemia, when further sustained, secondarily suppresses pancreatic insulin secretion and also reduces insulin action in the liver and muscle, thereby causing a vicious circle such as increased insulin resistance.

When insulin resistance persists, hyperinsulinemia itself decreases the number of insulin receptors or reduces the tyrosine kinase activity of the β-subunits of the receptors. As a result, hyperinsulinemia itself aggravates insulin resistance and further reduces the effect of insulin.

Oral antidiabetics that have been developed conventionally are as follows (Therapeutic Category: 396):
- 1.: insulin secretion-promoting agents:
- 1-a: sulfonylurea agents; tolbutamide (Hoechst Rastinon, etc), chlorpropamide (Diabinese, etc), acetohexamide (Dimelin), tolazamide (Tolinase), glyclopyramide (Deamelin-S), glibenclamide (Euglucon, Daonil, etc), gliclazide (Glimicron, etc)
- 1-b: sulfonylamide agents; glybuzole (Gludiase)
- 2.: insulin resistance-improving agents:
- 2-a: thiazolidine agents; troglitazone (Noscal (Rezulin)), pioglitazone
- 2-b: biguanide agents; buformin (Dibeton-S, etc), metformin (Glycoran, Melbin)
- 3.: postprandial hyperglycemia-improving agents:
- 3-a: α-glucosidase inhibitors; acarbose (Glucobay), voglibose (Basen).

These oral drugs for diabetes present a variety of problems. The sulfonylurea agents are drugs most commonly used for patients just diagnosed as having diabetes and however, accelerate pancreatic fatigue more than necessary unless exercise or diet therapy is sufficiently conducted on the patients. There is an indication that the influence of the α-glucosidase inhibitors on blood glucose levels is not sufficient.

On the other hand, thiazolidine derivatives, which unlike the sulfonylurea agents, are not mediated by the stimulation of insulin secretion, exhibit blood glucose-lowering action by enhancing insulin sensitivity in organisms, with the pancreatic β-cell function maintained; improving insulin resistance in insulin target organs accelerated in the state of diabetes; promoting glucose utilization in peripheral tissues; and suppressing glucose release in the liver.

The thiazolidine derivatives act on the pathway subsequent to the insulin binding of insulin receptors to ameliorate insulin resistance. In addition, they suppress glucose production in the liver and enhance glucose utilization in peripheral tissues, thereby lowering blood glucose. This action is probably achieved by normalizing the intracellular insulin signal transduction system that is a leading cause of insulin resistance. However, its molecular target is not quite elucidated.

Diabetes is a disease that results from the accumulation of plural gene mutations and environmental problems, and its root cause varies among individuals, even who develop similar symptoms. Genes known as the inheritance factors of diabetes are PPARγ, β3 adrenaline receptors, and adiponectin. Abnormalities in these three factors respectively make insulin resistance severe. The elucidation of molecular targets of antidiabetics is also important in developing tailor-made medical treatment based on patients' genetic information.

Non-Patent Document 1: WY FUJIMOTO "The importance of insulin resistance in the pathogenesis of type 2 diabetes mellitus." Am. J. Med., Apr 2000; 108 Suppl. 6a: 9S-14S

Non-Patent Document 2: M Diamant and RJ Heine "Thiazolidinediones in type 2 diabetes mellitus: current clinical evidence" Drugs, Jan 2003; 63(13): 1373-1405

### Disclosure of the Invention

Antidiabetics including thiazolidine derivatives and other agents exhibit high effectiveness against insulin resistance. However, under present circumstances, the mechanisms of their pharmacological actions are unknown. If the mechanisms of pharmacological actions of the antidiabetics such as thiazolidine derivatives, particularly molecular targets of thiazolidine derivatives, are elucidated, it is possible to develop novel drugs whose pharmacological actions are clear.

Thus, an object of the present invention is to elucidate a molecular target of an antidiabetic such as a thiazolidine derivative and provide a screening method for a novel antidiabetic, in consideration of the above-described situation.

The present inventors conducted intensive studies for attaining the object. As a result, the present inventors could identify a protein serving as a molecular target of the thiazolidine derivative and could develop a novel screening method using the protein, thereby completing the present invention.

Namely, the present invention encompasses the following inventions.
(1) A target protein of an antidiabetic, represented by the following (a) or (b):
   (a) a protein consisting of the amino acid sequence represented by SEQ ID NO: 2; or
   (b) a protein consisting of an amino acid sequence derived from the amino acid sequence represented by SEQ ID NO: 2 with the deletion, substitution, addition, or insertion of one or plural amino acids and interacting with the antidiabetic.
(2) The target protein according to (1), wherein the antidiabetic is a thiazolidine derivative.
(3) The target protein according to (2), wherein the thiazolidine derivative is pioglitazone.
(4) The target protein according to (1), wherein the target protein is a γ-tubulin ring complex protein.
(5) A gene encoding a target protein of an antidiabetic, represented by the following (a) or (b):
   (a) a protein consisting of the amino acid sequence represented by SEQ ID NO: 2; or
   (b) a protein consisting of an amino acid sequence derived from the amino acid sequence represented by SEQ ID NO: 2 with the deletion, substitution, addition, or insertion of one or plural amino acids and interacting with the antidiabetic.
(6) The gene encoding a target protein according to (5), wherein the antidiabetic is a thiazolidine derivative.
(7) The gene encoding a target protein according to (6), wherein the thiazolidine derivative is pioglitazone.
(8) The gene encoding a target protein according to (5), wherein the target protein is a γ-tubulin ring complex protein.
(9) A screening method for an antidiabetic, comprising the steps of:
   bringing a candidate substance to be screened into contact with a protein represented by the following (a) or (b):
      (a) a protein consisting of the amino acid sequence represented by SEQ ID NO: 2; or
      (b) a protein consisting of an amino acid sequence derived from the amino acid sequence represented by SEQ ID NO: 2 with the deletion, substitution, addition, or insertion of one or plural amino acids and interacting with the antidiabetic; and
   detecting the interaction between the candidate substance and the protein.
(10) The screening method for an antidiabetic according to (9), wherein the antidiabetic is a thiazolidine derivative.
(11) The screening method for an antidiabetic according to (10), wherein the thiazolidine derivative is pioglitazone.
(12) The screening method for an antidiabetic according to (9), wherein the target protein is a γ-tubulin ring complex protein.
(13) An antidiabetic screened by a screening method according to any one of (9) to (12) and mainly composed of a substance that interacts with the protein.
(14) A thiazolidine derivative represented by the general formula (I): 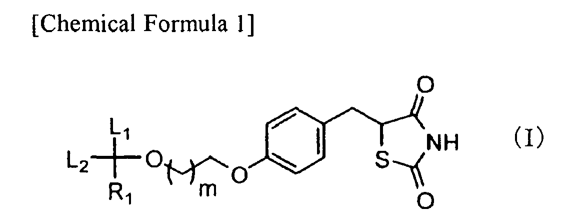 (in the formula (I), R₁ is hydrogen, a C₁₋₁₀ alkyl group, a C₃₋₇ cycloalkyl group, a C₇₋₁₁ phenylalkyl group, a phenyl group, or a five- or six-membered heterocyclic ring comprising 1 or 2 heteroatoms selected from the group consisting of nitrogen, oxygen, and sulfur; L₁ and L₂ are identical or different and are each independently hydrogen or a C₁₋₃ alkyl group or get together to form a C₂₋₆ cycloalkyl group; and m represents any integer from 1 to 5).
(15) The thiazolidine derivative according to (14), wherein in the formula (I), L₁ and L₂ get together to form a C₂₋₆ cycloalkyl group.
(16) The thiazolidine derivative according to (14), wherein in the formula (I), R₁ is hydrogen, and L₁ and L₂ get together to form a C₂₋₆ cycloalkyl group.
(17) The thiazolidine derivative according to (14), wherein in the formula (I), R₁ is a C₁₋₁₀ alkyl group, and L₁ and L₂ get together to form a C₂₋₆ cycloalkyl group.
(18) The thiazolidine derivative according to (14), wherein the thiazolidine derivative is 5-{4-[2-(1-methyl-cyclohexyloxy)-ethoxy]-benzyl}-thiazolidine-2,4-dione.
(19) A pharmacologically acceptable salt of a thiazolidine derivative according to any one of (14) to (18).
(20) A pharmaceutical composition comprising a thiazolidine derivative according to any one of (14) to (18) and/or a pharmacologically acceptable salt thereof as effective ingredients.
(21) The pharmaceutical composition according to (20), wherein the pharmaceutical composition is an antidiabetic.
(22) A process for manufacturing a thiazolidine derivative by subjecting, to condensation reaction, a compound represented by the general formula (II): 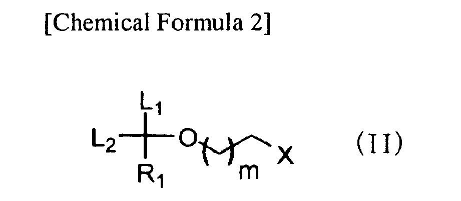 (in the formula (II), R₁ is hydrogen, a C₁₋₁₀ alkyl group, a C₃₋₇ cycloalkyl group, a C₇₋₁₁ phenylalkyl group, a phenyl group, or a five- or six-membered heterocyclic ring comprising 1 or 2 heteroatoms selected from the group consisting of nitrogen, oxygen, and sulfur; L₁ and L₂ are identical or different and are each independently hydrogen or a C₁₋₃ alkyl group or get together to form a C₂₋₆ cycloalkyl group; m represents any integer from 1 to 5; and X is one selected from the group consisting of MeSO₂, p-toluenesulfonyl, iodine, bromine, chlorine, and a hydroxy group) and
   a compound represented by the general formula (III):

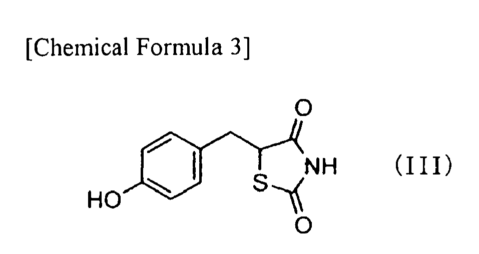

The present invention can provide a target protein that can be used in the screening of a novel antidiabetic, and a gene encoding the protein. The present invention can provide a screening method for an antidiabetic by using the target protein.

The present specification encompasses contents described in the specification and/or drawings of Japanese Patent Application No. 2003-402164 serving as a basis of the priority of the present application.

### Brief Description of the Drawings

Figure 1 is a characteristic chart showing a result of analyzing the interaction between pioglitazone and a FLJ14797-derived protein; and
Figure 2 is a diagram showing the alignment of the amino acid sequence represented by SEQ ID NO: 2 and the amino acid sequence of NP_055259.1.

### Best Mode for Carrying Out the Invention

Hereinafter, the present invention will be described in detail.

### Protein interacting with thiazolidine derivative

A protein according to the present invention is a protein that interacts with an antidiabetic. This protein interacts particularly with a thiazolidine derivative (concretely, pioglitazone), one of antidiabetics. The structural formula of pioglitazone is shown below.

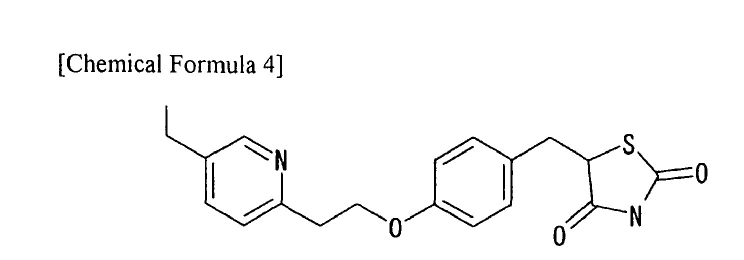

Examples of the antidiabetic that interacts with the protein according to the present invention can include a thiazolidine derivative. Examples of the thiazolidine derivative can include a compound having a structural formula analogous to the above-described structural formula and having pharmacological action similar to that of pioglitazone. However, the thiazolidine derivative with which the protein exhibits interaction is not limited to pioglitazone and can be exemplified by rosiglitazone, troglitazone, and ciglitazone.

The protein according to the present invention is, for example, a protein having the amino acid sequence represented by SEQ ID NO: 2. In this context, the protein according to the present invention is not limited to a protein consisting of the amino acid sequence represented by SEQ ID NO: 2 and also encompasses a protein consisting of an amino acid sequence derived from the amino acid sequence represented by SEQ ID NO: 2 with the substitution, deletion, insertion, or substitution of one or plural amino acids and interacting with the thiazolidine derivative.

The number and site of the substitution, deletion, or insertion of amino acids are not limited as long as its function is maintained. Concretely, the amino acid sequence may be derived from the amino acid sequence represented by SEQ ID NO: 2 with the substitution, deletion, or insertion of 1 to 30 amino acids, preferably 1 to 10 amino acids, more preferably 1 to 5 amino acids.

The protein according to the present invention may be a protein that is composed of an amino acid sequence with 50% or more homology, preferably 70% or more homology, more preferably 90% or more homology, to the amino acid sequence represented by SEQ ID NO: 2 and interacts with the thiazolidine derivative. In this context, the percent homology is determined by performing, for example, the commands of maximum matching method, in sequence analysis software DNASIS (Hitachi Software Engineering). Parameters used in this homology search are defaults (initial settings).

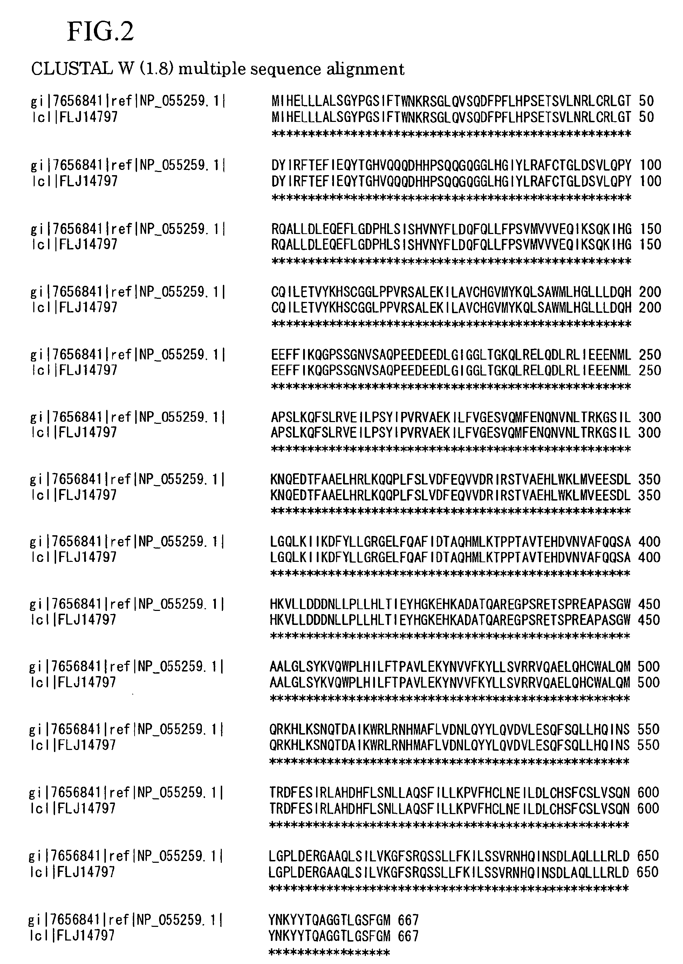

The protein represented by SEQ ID NO: 2 is encoded by cDNA registered as FLJ14797 in the NEDO (New Energy and Industrial Technology Development Organization) protein/cDNA structural analysis project (Http://www.nedo.go.jp/bip/) and exhibits high homology (93%) to Swiss-Prot Q9USQ2, GenBank AAH09870.1, and RefSeq NP_055259.1. The alignment of the amino acid sequence represented by SEQ ID NO: 2 and the amino acid sequence of NP_055259.1 is shown in Figure 2. AAH09870.1 and RefSeq NP_055259.1 are known as γ-tubulin ring complex protein GCP4 genes (Fava, F et al., Human 76p: A new member of the gamma-tubulin-associated protein family, J. Cell Biol. 147(4), 857-868 (1999)). Thus, the protein according to the present invention is also considered to be a protein that functionally has very high similarity to the γ-tubulin ring complex protein GCP4. Although a gene encoding the protein according to the present invention is disclosed in International Publication No. WO0204514, its function is unknown. It was not known until the disclosure of the present invention that the gene encodes the protein interacting with the thiazolidine derivative.

On the other hand, examples of the nucleotide sequence of the gene according to the present invention, that is, cDNA (FLJ14797) encoding the protein according to the present invention, can include, but not limited to, the nucleotide sequence represented by SEQ ID NO: 1. For example, the nucleotide sequence of the gene according to the present invention may be derived from the nucleotide sequence represented by SEQ ID NO: 1 whose codon is modified to encode the amino acid sequence represented by SEQ ID NO: 2. The gene according to the present invention also encompasses a gene comprising a nucleotide sequence that hybridizes under stringent conditions to a nucleotide sequence complementary to the nucleotide sequence encoding the amino acid sequence represented by SEQ ID NO: 1 and encodes the protein interacting with the thiazolidine derivative.

In this context, the phrase "hybridize under stringent conditions" means that a positive hybridization signal is still observed even under conditions of, for example, heating at 42°C in a solution of 6 × SSC, 0.5% SDS, and 50% formamide, followed by washing at 68°C in a solution of 0.1 × SSC and 0.5% SDS.

### Interaction between thiazolidine derivative and protein according to the present invention

Hereinafter, the interaction between the thiazolidine derivative and the protein according to the present invention will be described.

The causes of deficiencies in insulin action in diabetes are broadly divided into two mechanisms: reduction in the amount of insulin secreted from the pancreas; and reduction in insulin sensitivity in the liver or muscle (insulin resistance). Insulin action in the liver or muscle is based on an intracellular mechanism as illustrated below. Namely, the binding of insulin to an insulin receptor on the cell surface activates tyrosine kinase, which in turn causes the autophosphorylation of the insulin receptor. An intracellular substrate IRS-1 is bound to the phosphorylated tyrosine of the insulin receptor, which in turn phosphorylates the tyrosine of the IRS-1. PI3 kinase is then bound to the phosphorylated tyrosine of the IRS-1 and activated. The activated PI3 kinase phosphorylates PI to PI(3)P as well as PI(4)P to PI(3,4)P2 and PI(3,4,5)P3.

The intracellular signal through the PI3 kinase is deemed to be transmitted to, for example, the vesicle containing GLUT4, which is then translocated to the cell surface to promote the cellular uptake of glucose. On the other hand, Kapeller et al. (JBC, Vol. 270, pp. 25985-25991, 1995) and Inukai et al. (Biochem. J. Vol. 346, pp. 483-489, 2003) have reported that, though using human A431 cells or CHO cells in their experiments, PI3 kinase is bound to γ-tubulin by insulin stimulation. γ-tubulin, which contains small tubulin, is largely localized to the centrosome. The centrosome is the microtubule organizing center that is present in almost all animal cells and is located outside the nuclear membrane being contact thereto during the interphase of the cell cycle. The centrosome is doubled and divided into two parts during the interphase. At the start of mitosis, these two centrosomes migrate to the opposite sides of the nucleus to provide two poles of the spindle. Kapeller et al. suggests that PI3 kinase may influence the differentiation and proliferation of adipocytes and so on, via insulin-stimulated microtubule formation.

Because the protein according to the present invention, as described above, is considered to be a protein that functionally has very high similarity to the γ-tubulin ring complex protein GCP4, its binding with the thiazolidine derivative may be likely to enhance the binding between γ-tubulin and PI3 kinase.

On the other hand, ever since the possibility that thiazolidine derivatives serve as ligands of a peroxisome proliferator-activated receptor (PPAR)-γ was indicated, attention has been directed to how a mechanism works in which these insulin sensitivity-improving drugs exhibit pharmacological action via PPARγ. PPAR is one of nuclear hormone receptor superfamilies and forms a complex with retinoid-X-receptor-α (RXRα) when activated by ligand binding. This complex is bound as a transcription factor to a specific responsive element (peroxisome proliferator responsive element; PPRE) located upstream of a target gene to induce gene expression. The peroxisome proliferator (PP) responsible for the name of PPAR is a generic name for a group of chemical substances having common action of allowing an intracellular granule peroxisome to proliferate. PPAR, when originally found, was considered to be a receptor that mediates the pleiotropic effects (peroxisome proliferation, enzyme induction, and carcinogenesis) of PP. However, subsequent extensive studies including ligand search and target gene search have revealed that PPAR is an important regulator of many physiological functions including lipid metabolism.

PPAR has subtypes called PPARα, PPARβ (also called PPARδ or NUC1), and PPARγ (classified as PPARγ1 and PPARγ2 according to differences in transcription initiation sites and alternative splicing). The ability of some chemical substance (ligand) to activate PPAR differs depending on each subtype, and the expression of each subtype differs from one tissue to another. The expression of PPARα is high in the liver, cardiac muscle, intestine, and proximal renal tubule. PPARβ is expressed in a wide range of tissues and is sometimes expressed at levels higher than those of the subunits α and γ. PPARγ is mainly expressed in adipose tissues and the immune system. Such topographical variations suggest that the PPAR subtypes have different physiological roles.

As target genes of PPAR have been elucidated, PPARα has been shown to control the expression of a variety of genes involved in lipid oxidation mainly in the liver and cardiac muscle, and so on. On the other hand, PPARγ is highly expressed in adipose tissues and has function as a transcription factor during terminal adipocyte differentiation in white adipose tissues. PPARγ2 has been cloned as a component of ARF-6, a specific transcription factor that transactivates an adipocyte fatty acid-binding protein aP2. Moreover, a PPARγ2/RXRα heterodimer induces the adipocyte-specific expression of phosphoenolpyruvate carboxykinase(PEPCK) to generate glycerol. Both aP2 and PEPCK genes are indicators for terminal adipocyte differentiation. The control of these genes by PPARγ indicates that this receptor plays an important role in maintaining adipocyte phenotypes. However, an evident mechanism that directly links thiazolidine derivatives with PPARγ is still largely unknown. Moreover, heterozygous CBP (cAMP response element binding protein (CREB)-binding protein)-deficient mice exhibit antiobesity and antidiabetes phenotypes more strongly than heterozygous PPARγ-deficient mice, suggesting the presence of a novel PPARγ-independent signal transduction pathway with antiobesity and antidiabetes effects.

On the other hand, a deficiency in adiponectin discovered as a gene product highly and specifically expressed in adipocyte tissues is also considered to be one of important causes of insulin resistance in obesity and type 2 diabetes. This idea is based on the findings that adiponectin is hyperexpressed in heterozygous PPARγ-deficient mice favorably sensitive to insulin and that adiponectin genes are primary disease-sensitive genes of type 2 diabetes in the Japanese. Adiponectin is primary insulin-sensitive hormone derived from white adipocytes, and the replenishment of adiponectin improves insulin resistance in lipoatrophic diabetes. In patients with type 2 diabetes, it is conceivable that a deficiency in adiponectin elicits insulin resistance, while the replenishment of adiponectin improves insulin resistance. Alternatively, because homozygous adiponectin-deficient mice exhibit reduced glucose tolerance, adiponectin is expected to act as an *in-vivo* antidiabetes factor. Furthermore, the administration of adiponectin increases the expression of factors involved in fatty acid combustion and energy spending, in experiments of adiponectin administration to insulin-resistant lipoatrophic diabetes mice or KKAy mice or in experiments of crossbreeding between ob/ob mice (obese, insulin-resistant model mice) and adiponectin-overexpressing transgenic mice. It has been reported that this may be due to increases in the expression level of PPARα targeted for these genes and in the endogenous ligand activity itself of PPARα by the administration of adiponectin (Yamauchi et al., "Advances in Molecular Diabetology 2003 -From Basic Research to the Clinic-" Kanehara-Shuppan, pp. 97-106).

Meanwhile, Surapureddi et al. (PNAS, Vol. 99, pp. 11836-11841, 2002) have found in assay using rat liver tissue extracts that coactivators called as PRIC complexes bind to PPARα to enhance its function, and have further found in pull-down assay using GST-tagged PPARα as a bait that among the PRIC complexes, a tubulin-binding protein called TOG binds to PPARα. Moreover, Spittle et al. (JBC, Vol. 275, pp. 20748-20753, 2000) have reported that TOG proteins bind to a microtubule structure very similar to the γ-tubulin ring complex.

From these reports and the results by the present inventors, it is conceivable that pioglitazone may mimic the action of adiponectin and improve insulin resistance in patients with type 2 diabetes, by enhancing the interaction among those three factors, γ-tubulin ring complex, TOG, and PPARα, via its binding with the γ-tubulin ring complex. Alternatively, on the assumption that for PPARγ, there would exist a protein analogous to the TOG protein, it is also conceivable that pioglitazone may promote the interaction among three factors, γ-tubulin ring complex, TOG, and PPARγ, to activate PPARγ.

As described above, the present inventors found that the γ-tubulin ring complex is one of molecular targets of the thiazolidine derivative, and that the thiazolidine derivative may improve insulin sensitivity by enhancing the signal of PI3 kinase and/or the action of PPARα via its binding with the γ-tubulin ring complex and exhibit therapeutic effect on insulin resistance.

### Screening method using protein according to the present invention

As described above, the protein according to the present invention can be used to establish a screening method for a novel antidiabetic by utilizing the interaction between the thiazolidine derivative and the protein according to the present invention.

The screening method comprises the steps of: bringing a candidate substance to be screened into contact with the protein according to the present invention; and detecting the presence or absence of the interaction between the candidate substance and the protein.

Examples of the candidate substance to be screened can include, but not particularly limited to, a variety of low molecular weight compounds and proteins.

Any method known as an analytical method of the interaction between two molecules, a candidate substance and an object substance, can be used without particular limitations as a method for bringing the candidate substance to be screened into contact with the protein according to the present invention. Examples thereof can include: a method that utilizes an apparatus (eg., Biacore 3000; manufactured by Biacore) for performing interaction analysis in real time by applying the surface plasmon resonance (SPR) principle; and an approach that employs chromatography as an approach for analyzing the interaction between two molecules without modification and immobilization (U.S. Patent No. 4,762,617). In addition, methods described in Y. Dunayevskiy et al., Rapid Comm. Mass Spectrometry, vol. 11, 1178-1184 (1997), in F.J. Moy et al., Anal. Chem., vol. 73, 571-581 (2001), in International Publication No. WO 00/47999, and in JP Patent Publication (Kohyo) NOs. 2002-508515A (2002) and 2003-502665A (2003) can be utilized as appropriate.

Alternative methods can also be used as appropriate, which include other surface plasmon resonance techniques such as quartz resonator method, coupled waveguide surface plasmon resonance method, dual polarization interferometry, calorimetry, centrifugal sedimentation method, capillary electrophoresis, energy transfer method, fluorescence polarization method, fluorescence correlation spectroscopy, protein chips, and compound chips.

The candidate substance judged as having interaction with the protein according to the present invention by the screening method of the present invention means that the substance has been screened as a novel antidiabetic having the mechanism of pharmacological action similar to that of the thiazolidine derivative.

### Screened novel compound

A thiazolidine derivative represented by the general formula (I) below was identified as a candidate substance of an antidiabetic by the screening method. The compound represented by the general formula (I) below is a novel substance. 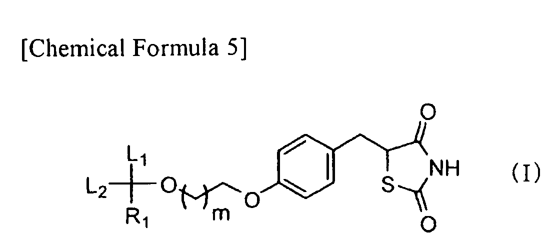 In the formula (I), R₁ is hydrogen, a C₁₋₁₀ alkyl group, a C₃₋₇ cycloalkyl group, a C₇₋₁₁ phenylalkyl group, a phenyl group, or a five- or six-membered heterocyclic ring comprising 1 or 2 heteroatoms selected from the group consisting of nitrogen, oxygen, and sulfur; L₁ and L₂ are identical or different and are each independently hydrogen or a C₁₋₃ alkyl group or get together to form a C₂₋₆ cycloalkyl group; and m represents any integer from 1 to 5.

Concrete examples of the compound represented by the general formula (I) can include 5-{4-[2-(1-methyl-cyclohexyloxy)-ethoxy]-benzyl}-thiazolidine-2,4-dione represented by the formula A-1 below.

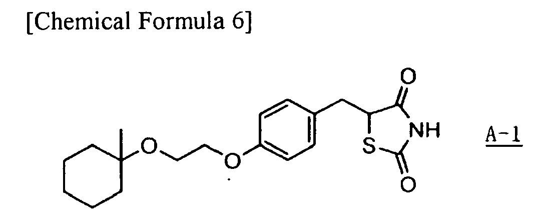

The compound represented by the formula (I) is not limited to the compound A-1 and can be exemplified by compounds A-2 and A-3 below.

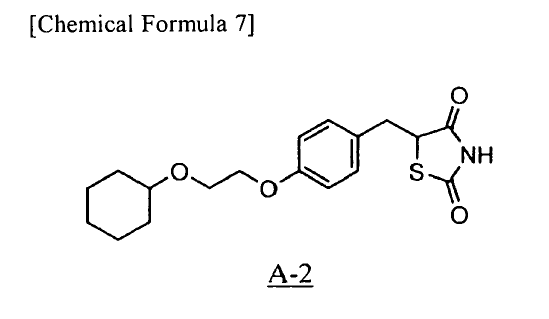

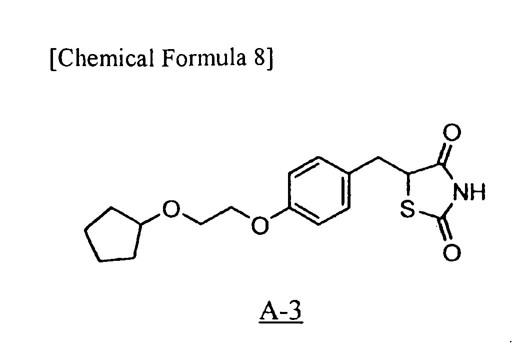

The compound represented by the formula (I) can be manufactured by subjecting, to condensation reaction, a compound represented by the following formula (II): 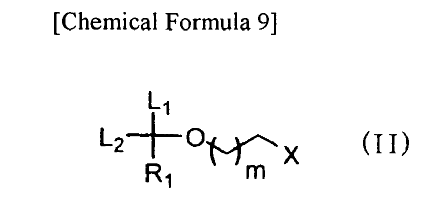 (in the formula (II), the definitions of R₁, L₁, and L₂ are the same as in the formula (I); and X is one selected from the group consisting of MeSO₂, p-toluenesulfonyl, iodine, bromine, chlorine, and a hydroxy group) and
a compound represented by the following formula (III):

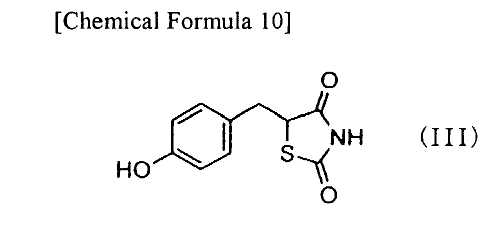

By way of example, a process for manufacturing the compound represented by the formula A-1 will be described. At first, (1-methyl-cyclohexyl)-tert-butyl acetate (2) is synthesized as illustrated below.

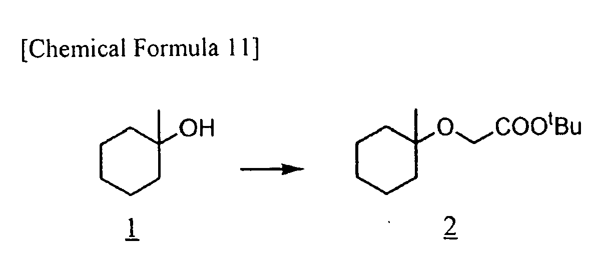

Specifically, the compound (1) (713 mg, 6.2 mmol) is gradually added with stirring to 40 mL of an ice-cold THF/DMF (5/1) mixture suspension containing sodium borohydride (in oil; 60 wt%, 300 mg) under a nitrogen atmosphere and then stirred for 10 minutes. Subsequently, the ice bath is removed, and the resulting reaction solution is stirred at room temperature for 30 minutes. After the completion of stirring, the reaction solution is ice-cold again. Next, t-butyl bromoacetate (2.25 mL, 15.5 mmol) is added thereto. After the completion of addition, the ice bath is removed, and the resulting reaction solution is stirred at room temperature for 2 hours. The reaction solution is then ice-cold again, and water (1 mL) is gradually added to the reaction solution. Water and ethyl acetate are poured to the reaction solution and stirred well, followed by the collection of the organic phase. After extraction from ethyl acetate, the organic phase is collected and dried over anhydrous sodium sulfate. After filtration and concentration under reduced pressure, the compound (2) (¹H-NMR (CDCl₃) δ: 1.28-1.38 (13H, m), 1.40 (9H, s), 4.02 (2H, s)) can be synthesized by purifying the concentrate with a silica gel column chromatograph.

Next, 2-(1-methyl-cyclohexyl)-ethanol (3) is synthesized from the compound (2) as illustrated below.

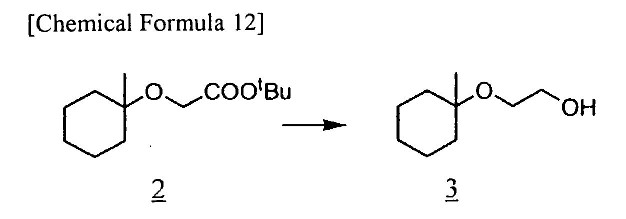

Specifically, 2 mL of a THF solution of the compound (2) (254 mg, 2 mmol) is gradually added with stirring to 4 mL of an ice-cold THF suspension containing lithium aluminum hydride (304 mg, 8 mmol) under a nitrogen atmosphere. After the completion of addition, the reaction container is heated to 60°C and stirred for 2 hours. After the completion of stirring, the reaction solution is ice-cold again, and a saturate sodium sulfate solution is gradually added to the reaction solution until no hydrogen is generated. The residue is filtered on cerite and washed with ethyl acetate. The filtrate is combined with a washing liquid and concentrated under reduced pressure. The compound (3) (¹H-NMR (CDCl₃) δ: 1.28-1.38 (13H, m), 3.50-3.75 (4H, m)) can be synthesized by purifying the semi-purified product with a silica gel column chromatograph.

Next, 5-{4-[2-(1-methyl-cyclohexyloxy)-ethoxy]-benzyl}-thiazolidine-2,4-dione (A-1) is synthesized from the compound (3) as illustrated below.

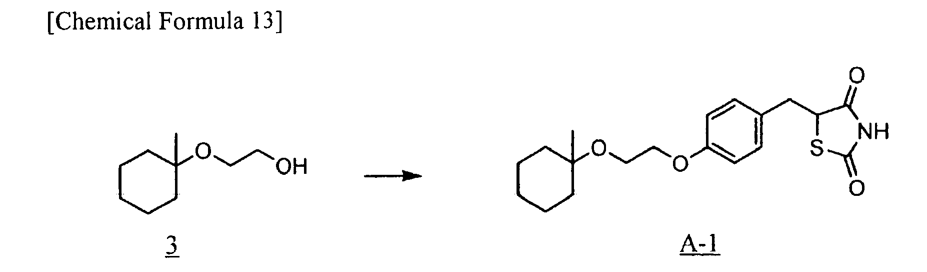

Specifically, tributyl phosphine (234 mg, 1.1 mmol) is added to a toluene (2 mL) solution of the compound (3) (158 mg, 1.0 mmol) under a nitrogen atmosphere and stirred for 20 minutes. Subsequently, this solution is added at room temperature to a toluene (2 mL) solution containing 5-(4-hydroxy-benzyl)-thiazolidine-2,4-dione (246 mg, 1.1 mmol) and 1,1'-azobis(N,N'-dimethylformamide) (200 mg, 1.1 mmol) and stirred overnight. After the completion of stirring, ethyl acetate is further added thereto. The residue is filtered on cerite and further washed with ethyl acetate. The filtrate is combined with a washing liquid. After concentration under reduced pressure, the compound (A-1) (¹H-NMR (CDCl₃) δ: 1.28-1.38 (13H, m), 3.46 (2H, d), 3.79 (2H, t), 4.11 (2H, t), 4.13 (1H, t), 6.72 (2H, d), 7.00 (2H, d)) can be synthesized by purifying the semi-purified product with a silica gel column chromatograph.

The compound A-2 (¹H-NMR (CDCl₃) δ: 1.28-1.42 (10H, m), 2.85 (1H, m), 3.46 (2H, d), 3.79 (2H, t), 4.11 (2H, t), 4.13 (1H, t), 6.70 (2H, d), 7.00 (2H, d)) can be synthesized by performing each reaction in the same way as in the above-described synthesizing process except that cyclohexanol is used as a starting material instead of the compound (1). Alternatively, the compound A-3 (¹H-NMR (CDCl₃) δ: 1.51-1.60 (8H, m), 2.85 (1H, m), 3.46 (2H, d), 3.79 (2H, t), 4.11 (2H, t), 4.10 (1H, t), 6.72 (2H, d), 7.00 (2H, d)) can be synthesized by performing each reaction in the same way as in the above-described synthesizing process except that cyclopentanol is used as a starting material.

On the other hand, the novel compound represented by the general formula (I) may be used in the form of a pharmacologically acceptable salt. Examples of the "pharmacologically acceptable salt" can include a salt of an inorganic acid such as hydrochloric acid, sulfuric acid, nitric acid, or phosphoric acid; a salt of an organic acid such as para-toluenesulfonic acid, methanesulfonic acid, oxalic acid, or citric acid; a salt of an organic base such as ammonium, trimethylammonium, or triethylammonium; a salt of alkali metal such as sodium or potassium; a quaternary salt with alkyl halide such as methyl iodide or ethyl iodide; and a salt of alkaline-earth metal such as calcium or magnesium. The novel compound (I) is meant to encompass a hydrate thereof, and any number of water molecules may be coordinated for the compound (I). Moreover, the novel compound (I) is meant to encompass a prodrug thereof. The prodrug refers to a derivative of the novel compound (I) having a group metabolically degraded *in vivo,* and this derivative is a compound that exerts its pharmacological action after being converted to the novel compound (I) through the *in-vivo* metabolic process. Methods for selecting and manufacturing an appropriate prodrug derivative are described in, for example, Design of Prodrugs, Elsevier, Amsterdam 1985.

For example, when the novel compound (I) has a carboxy group, the prodrug according to the present invention is exemplified by a prodrug such as an ester derivative produced by the reaction between the carboxy group and appropriate alcohol or an amide derivative produced by the reaction between the carboxy group and appropriate amine. For example, when the novel compound (I) has a hydroxy group, it is exemplified by a prodrug such as an acyloxy derivative produced by the reaction between the hydroxy group and an appropriate acyl halide or acid anhydride. For example, when the novel compound (I) has an amino group, it is exemplified by a prodrug such as an amide derivative produced by the reaction between the amino group and an appropriate acid halide or mixed acid anhydride.

When the novel compound (I) has an asymmetric carbon atom, the present invention encompasses a racemic body, both enantiomorphs, and all stereoisomers (diastereoisomers). When the novel compound (I) has a double bond, the present invention encompasses all geometric isomers, if any, in each substituent configuration of the double bond.

The novel compound (I) as described above is used as a pharmaceutical composition having action as a novel antidiabetic. When the pharmaceutical composition is administered as an antidiabetic, the administration can be performed by both oral and parenteral methods. For oral administration, the pharmaceutical composition may be prepared according to a routine method into a dosage form typically used such as a tablet, granule, powder, capsule, pill, liquid medicine, syrup, buccal, or sublingual tablet. For parenteral administration, the pharmaceutical composition can be administered preferably in any dosage form typically used such as an injection for intramuscular or intravenous administration, a suppository, percutaneously absorbable agent, or inhalant.

Moreover, a pharmaceutical preparation can be produced by mixing a variety of pharmaceutical additives such as an excipient, binder, wetting agent, disintegrant, lubricant, and diluent suitable for the dosage form with an effective amount of the pharmaceutical composition as necessary. The preparation may be produced by performing sterilization treatment together with an appropriate carrier, when used in the form of an injection. Concrete examples of the pharmaceutical additives include: milk sugar, white sugar, grape sugar, starch, calcium carbonate, or crystalline cellulose as the excipient; methylcellulose, carboxymethylcellulose, hydroxypropylcellulose, gelatin, or polyvinylpyrrolidone as the binder; carboxymethylcellulose, carboxymethylcellulose sodium, starch, sodium alginate, agar powder, or sodium lauryl sulfate as the disintegrant; and talc, magnesium stearate, or macrogol as the lubricant. For example, cocoa butter, macrogol, or methylcellulose can be used as a base for a suppository. When the pharmaceutical preparation is prepared as a liquid medicine or an emulsifiable or suspensible injection, a solubilizing agent, suspending agent, emulsifier, stabilizer, preservative, isotonic agent, and so on, typically used may be added as appropriate. For oral administration, a flavoring agent, aromatic substance, and so on may be added thereto.

Desirably, the dose of the novel compound (I) used as an antidiabetic is decided in consideration of the age and body weight of a patient, the type and severity of disease, an administration route, and so on. The novel compound (I) is orally administered to an adult at a dose that falls within a range of typically 1 to 100 mg/kg/day, preferably 5 to 30 mg/kg/day. Alternatively, the novel compound (I) is parenterally administered to an adult at a dose that falls within a range of typically 0.1 to 10 mg/kg/day, preferably 1 to 5 mg/kg/day, although the dose largely varies depending on an administration route. The novel compound (I) within this range may be administered at one dose or several divided doses per day.

### Examples

Hereinafter, the present invention will be described more fully with reference to Examples. The technical scope of the present invention is not intended to be limited to Examples below.

### [Reference Example 1]

### Method for protein expression from human full-length cDNA clones

### 1. Preparation of expression plasmids

Genes of interest in human full-length cDNA clones were subjected to BP reaction with a PCR cloning vector Gateway pDONR201 using Gateway system available from Invitrogen according to the kit's protocol to give an entry vector. pEU3-NII (TOYOBO) compatible with a cell-free protein synthesis system (PROTEIOS; TOYOBO) using wheat germ extracts was used as a source vector, from which a double-tag destination vector used as the destination vector of the Gateway system was prepared by introducing Gateway cassette with Gateway recombinant sequence into the pEU3-NII vector so that the Gateway system could be utilized, and further modifying the resulting vector by PCR so that peptides having histidine and FLAG tag sequences would be expressed in the N-terminal region of an expressed protein.

The prepared double-tag destination vector and entry vector were used to conduct BP reaction using the Gateway system (Invitrogen) according to the protocol. The resulting product was transformed into *Escherichia coli* competent cells DH5α to select clones where the expression vector was introduced. Plasmids were prepared from the obtained clones using QIAfilter Midi kit (QIAGEN) according to the kit's protocol. The obtained plasmids were treated with phenol and chloroform according to the PROTEIOS (TOYOBO) protocol and subjected to the inactivation treatment of RNase to give purified expression plasmids.

### 2. Acquisition of purified proteins

Recombinant proteins were synthesized by the cell-free protein synthesis system (PROTEIOS; TOYOBO) using wheat germ extracts. mRNA was prepared according to the PROTEIOS protocol from the expression plasmids obtained by the method described in the paragraph 1. A 20-µg aliquot of the obtained mRNA was used to synthesize proteins in 2 wells of a 96-well micro titer plate according to the PROTEIOS protocol. The synthesized proteins were subjected to high-speed centrifugation treatment to remove precipitations. The obtained soluble fractions were purified using ANTI-FLAG M2 Affinity Gel (SIGMA) immobilizing thereon an anti-FLAG tag antibody according to the protocol to give purified proteins.

### [Reference Example 2]

### Method for determining binding dissociation constant in human protein-pharmaceutical drug interaction using Biacore

The surface of a CM5 sensor chip for S51 commercially available from Biacore was converted to NTA using 1M EDC, 1.33 M NHS, and 16mg/ml AB-NTA (pH 9.2) to make an NTA sensor chip for S51. The proteins expressed in the wheat germ system and purified with a FLAG tag were immobilized on this chip. The immobilization was performed by sequentially injecting 0.5 M NiCl₂, 0.4 M EDC, 0.1 M EDC, a ligand (protein) solution, and 1 M ethanolamine (pH 8.5) into the passage system of Biacore S51. A running buffer used for the immobilization was PBS (pH 7.4). The ligand-immobilized sensor chip was used to conduct assay described below. A running buffer used was prepared by adding DMSO at the final concentration of 5% to HBS (10 mM HEPES and 150 mM NaCl, (pH 7.6)), 0.005% P20, and 100 uM mineral ion cocktail (Ca(OAc)₂, Zn(OAc)₂.2H₂O, Cu(OAc)₂.H₂O, Co(OAc)₂.4H₂O, Mn(OAc)₂.4H₂O, Mg(OAc)₂.4H₂O, and FeCl₃.6H₂O). Compounds to be measured were prepared by making 1/2 serial dilutions (9 points) from 62.5 uM to 0.244 uM solutions. Solvents used for the compound solutions were prepared in the same composition as that of the running buffer. A solution containing only the solvents without the compound was prepared for zero-concentration measurement. For the correction (solvent correction) of the effect of DMSO contained in the compound solutions and the running buffer, the same solutions as the running buffer containing 3.8 to 5.1% DMSO (8 points) were prepared to perform the correction on the basis of measurement results of these solutions. The Compound Characterization Assay program of Biacore S51 was conducted to measure the interaction between the immobilized ligands (proteins) and the analytes (compounds; 62.5 uM to 0.244 uM), followed by analysis with specific software.

### [Example 1]

### Analysis of interaction between pioglitazone and FLJ14797-derived protein

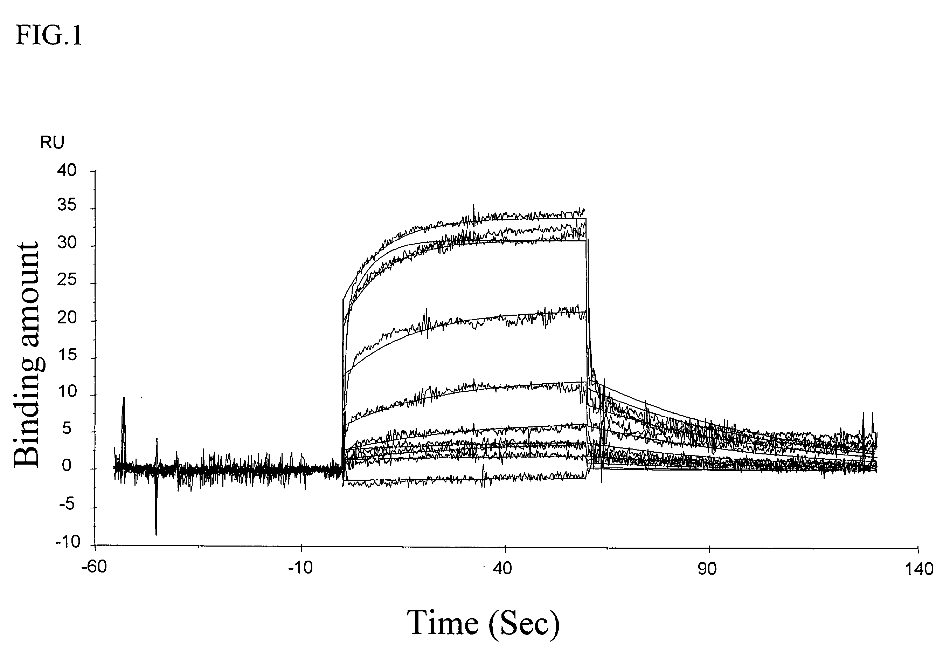

Proteins were expressed and purified from FLJ14797 according to the method of Reference Example 1, while the interaction between pioglitazone and the protein expressed and purified from FLJ14797 was analyzed according to the method of Reference Example 2. The result was shown in Figure 1. The binding amount was increased dose-dependently on pioglitazone, and the binding was observed to be saturated at high doses of pioglitazone. Therefore, the interaction between them was confirmed to be specific. A binding dissociation constant calculated using the Biacore S51 specific software was KD = 9.038 × 10⁻⁶ M.

The result shown in Figure 1 demonstrated that pioglitazone interacts with FLJ14797-derived proteins. Thus, the FLJ14797-derived proteins were found to be target proteins of pioglitazone (thiazolidine derivative) known as an antidiabetic. As seen from these results, a novel antidiabetic can be screened by allowing the FLJ14797-derived protein to act on a candidate substance to be screened. Namely, the screening of a novel antidiabetic can be performed by constructing such a system as to detect the interaction between the FLJ14797-derived protein and the candidate substance by, for example, the method of Reference Example 2.

### [Example 2]

### Analysis of interaction between novel compound (I) and FLJ14797-derived protein

The interaction between the FLJ14797-derived protein and each compound was analyzed in the same way as in Example 1 except that the above-described compounds A-1, A-2, and A-3 were used instead of pioglitazone used in Example 1. KD values calculated in the same way as in Example 1 are shown in Table 1.

**[Table 1]**

| Compound | KD (M) |
|---|---|
| A-1 | 8.431 × 10⁻⁶ M |
| A-2 | 1.382 × 10⁻⁵ M |
| A-3 | 2.156 × 10⁻⁵ M |

As seen from Table 1, specific interaction with the FLJ14797-derived protein was observed for all the compounds. This result demonstrated that the compound represented by the general formula (I) interacts with the FLJ14797-derived protein, that is, the γ-tubulin ring complex protein.

All publications, patents, and patent applications cited herein are incorporated herein by reference in their entirety.

## Claims

1. A target protein of an antidiabetic, represented by the following (a) or (b):
(a) a protein consisting of the amino acid sequence represented by SEQ ID NO: 2; or
(b) a protein consisting of an amino acid sequence derived from the amino acid sequence represented by SEQ ID NO: 2 with the deletion, substitution, addition, or insertion of one or plural amino acids and interacting with the antidiabetic.

2. The target protein according to claim 1, wherein the antidiabetic is a thiazolidine derivative.

3. The target protein according to claim 2, wherein the thiazolidine derivative is pioglitazone.

4. The target protein according to claim 1, wherein the target protein is a γ-tubulin ring complex protein.

5. A gene encoding a target protein of an antidiabetic, represented by the following (a) or (b):
(a) a protein consisting of the amino acid sequence represented by SEQ ID NO: 2; or
(b) a protein consisting of an amino acid sequence derived from the amino acid sequence represented by SEQ ID NO: 2 with the deletion, substitution, addition, or insertion of one or plural amino acids and interacting with the antidiabetic.

6. The gene encoding a target protein according to claim 5, wherein the antidiabetic is a thiazolidine derivative.

7. The gene encoding a target protein according to claim 6, wherein the thiazolidine derivative is pioglitazone.

8. The gene encoding a target protein according to claim 5, wherein the target protein is a γ-tubulin ring complex protein.

9. A screening method for an antidiabetic, comprising the steps of:
bringing a candidate substance to be screened into contact with a protein represented by the following (a) or (b):
(a) a protein consisting of the amino acid sequence represented by SEQ ID NO: 2; or
(b) a protein consisting of an amino acid sequence derived from the amino acid sequence represented by SEQ ID NO: 2 with the deletion, substitution, addition, or insertion of one or plural amino acids and interacting with the antidiabetic; and
detecting the interaction between the candidate substance and the protein.

10. The screening method for an antidiabetic according to claim 9, wherein the antidiabetic is a thiazolidine derivative.

11. The screening method for an antidiabetic according to claim 10, wherein the thiazolidine derivative is pioglitazone.

12. The screening method for an antidiabetic according to claim 9, wherein the target protein is a γ-tubulin ring complex protein.

13. An antidiabetic screened by a screening method according to any one of claims 9 to 12 and mainly composed of a substance that interacts with the protein.

14. A thiazolidine derivative represented by the general formula (I): 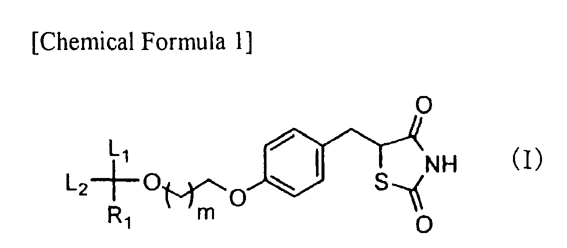 (in the formula (I), R₁ is hydrogen, a C₁₋₁₀ alkyl group, a C₃₋₇ cycloalkyl group, a C₇₋₁₁ phenylalkyl group, a phenyl group, or a five- or six-membered heterocyclic ring comprising 1 or 2 heteroatoms selected from the group consisting of nitrogen, oxygen, and sulfur; L₁ and L₂ are identical or different and are each independently hydrogen or a C₁₋₃ alkyl group or get together to form a C₂₋₆ cycloalkyl group; and m represents any integer from 1 to 5).

15. The thiazolidine derivative according to claim 14, wherein in the formula (I), L₁ and L₂ get together to form a C₂₋₆ cycloalkyl group.

16. The thiazolidine derivative according to claim 14, wherein in the formula (I), R₁ is hydrogen, and L₁ and L₂ get together to form a C₂₋₆ cycloalkyl group.

17. The thiazolidine derivative according to claim 14, wherein in the formula (I), R₁ is a C₁₋₁₀ alkyl group, and L₁ and L₂ get together to form a C₂₋₆ cycloalkyl group.

18. The thiazolidine derivative according to claim 14, wherein the thiazolidine derivative is 5-{4-[2-(1-methyl-cyclohexyloxy)-ethoxy]-benzyl}-thiazolidine-2,4-dione.

19. A pharmacologically acceptable salt of a thiazolidine derivative according to any one of claims 14 to 18.

20. A pharmaceutical composition comprising a thiazolidine derivative according to any one of claims 14 to 18 and/or a pharmacologically acceptable salt thereof as effective ingredients.

21. The pharmaceutical composition according to claim 20, wherein the pharmaceutical composition is an antidiabetic.

22. A process for manufacturing a thiazolidine derivative by subjecting, to condensation reaction, a compound represented by the general formula (II): 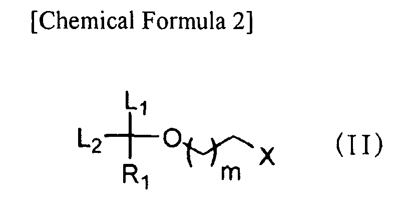 (in the formula (II), R₁ is hydrogen, a C₁₋₁₀ alkyl group, a C₃₋₇ cycloalkyl group, a C₇₋₁₁ phenylalkyl group, a phenyl group, or a five- or six-membered heterocyclic ring comprising 1 or 2 heteroatoms selected from the group consisting of nitrogen, oxygen, and sulfur; L₁ and L₂ are identical or different and are each independently hydrogen or a C₁₋₃ alkyl group or get together to form a C₂₋₆ cycloalkyl group; m represents any integer from 1 to 5; and X is one selected from the group consisting of MeSO₂, p-toluenesulfonyl, iodine, bromine, chlorine, and a hydroxy group) and
a compound represented by the general formula (III):
